# EUROPÄISCHE PATENTSCHRIFT

(11) **EP 2 401 078 B1**
(45) Veröffentlichungstag und Bekanntmachung des Hinweises auf die Patenterteilung: **23.10.2013**
(21) Anmeldenummer: 10711629.5
(22) Anmeldetag: 23.02.2010
(51) Int. Cl.: B01J 31/40, B01J 31/18

(54) **VERFAHREN ZUR ANREICHERUNG EINES HOMOGENKATALYSATORS AUS EINEM PROZESSSTROM**
METHOD FOR ENRICHING A HOMOGENOUS CATALYST FROM A PROCESS FLOW
PROCÉDÉ D'ENRICHISSEMENT D'UN CATALYSEUR HOMOGÈNE ISSU D'UN FLUX DE PROCESSUS

(30) Priorität: 27.02.2009 DE 102009001225
(43) Veröffentlichungstag der Anmeldung: 04.01.2012
(73) Patentinhaber: Evonik Degussa GmbH, 45128 Essen (DE)
(72) Erfinder: PRISKE, Markus, 40472 Düsseldorf (DE); BAUMGARTEN, Götz, 45721 Haltern am See (DE); LÜKEN, Hans-Gerd, 45770 Marl (DE); KAIZIK, Alfred, 45772 Marl (DE); WIESE, Klaus-Diether, 45721 Haltern am See (DE); ERNST, Uwe, 45770 Marl (DE); MUHLACK, Patrick, 45659 Recklinghausen (DE)
(86) Internationale Anmeldenummer: PCT/EP2010/052242
(87) Internationale Veröffentlichungsnummer: WO 2010/097376

(56) Entgegenhaltungen:
- EP-A1- 0 850 905
- WO-A1-03/095402
- WO-A1-03/095406
- DE-A1- 19 801 437
- DE-A1-102005 046 250
- DE-A1-102005 060 784
- DE-A1-102006 003 618
- MCKEOWN N B ET AL: "Polymers of intrinsic microporosity (PIMs): Bridging the void between microporous and polymeric materials" CHEMISTRY - A EUROPEAN JOURNAL, WILEY - V C H VERLAG GMBH & CO. KGAA, WEINHEIM, DE LNKD- DOI:10.1002/CHEM.200400860, Bd. 11, Nr. 9, 22. April 2005 (2005-04-22) , Seiten 2610-2620, XP002493889 ISSN: 0947-6539

## Beschreibung

Die vorliegende Erfindung betrifft ein Verfahren zur Anreicherung eines homogenen Katalysators aus einem Prozessstrom, der diesen Homogenkatalysator als Bestandteil enthält. Zur Anreicherung wird der Prozessstrom über mindestens eine Membran geführt.

In der homogenen Katalyse eingesetzte Katalysatorsysteme müssen in der Regel aus dem jeweiligen Reaktionsgemisch ganz oder teilweise entfernt werden. Gründe hierfür können Anforderungen an die Produktreinheit sein oder die Rückgewinnung des Katalysatorsystems als Wertstoff, welcher z. B. in die Reaktion direkt oder indirekt zurückgeführt werden kann.

Insbesondere beim Einsatz von Übergangsmetallkomplexen, wie z. B. Rhodium als Katalysator oder beim Einsatz teurer Liganden stellt die Abtrennung des Katalysatorsystems aufgrund der Katalysatorkosten einen wichtigen Prozessschritt dar. Technische Verfahren, die unter Verwendung von Übergangsmetallkomplexen in homogener Phase durchgeführt werden, sind beispielsweise Telomerisierungen, Metathesen, Hydrierungen oder Hydroformylierungen. Technisch verbreitet und mit relativ hohen Katalysatorkosten verbunden, sind Reaktionen mit Rhodiumkomplexen.

Rhodiumkomplexe werden als Katalysator z. B. bei der großtechnischen Hydroformylierung von Olefinen zu den um ein C-Atom reicheren Aldehyden und/oder Alkoholen verwendet. Dabei werden insbesondere Rhodiumkomplexe mit Phosphin-, Phosphit-, Phosphonit- oder Phosphinitliganden als Katalysator eingesetzt.

Die Art der Abtrennung kann einen wesentlichen Einfluss auf die Wirtschaftlichkeit des Gesamtprozesses besitzen. Die Abtrennung des Katalysators vom Reaktionsgemisch kann auf einfachste Weise ausschließlich durch thermische Trennverfahren erfolgen, beispielsweise indem das Reaktionsprodukt und gegebenenfalls Edukt aus dem katalysatorhaltigen Reaktionsgemisch durch Verdampfen abgetrennt wird. Nachteilig bei solchen Verfahren ist, dass der Katalysator und/oder der Ligand sich während der Destillation zersetzen können. Die Katalysatorfolgeprodukte im Destillationsrückstand können häufig im Prozess nicht in ein aktives Katalysatorsystem umgewandelt werden. Sie müssen daher ausgeschleust und vor Rückführung in den Prozess aufwändig aufgearbeitet werden. Dies gilt insbesondere für die Aufarbeitung von Hydroformylierungsgemischen, die als Katalysatoren Rhodiumkomplexe mit Liganden, die das Rhodium schwächer als Phosphine komplexieren, enthalten. Bei der Destillation können sich diese Ligandkomplexe wegen der fehlenden Stabilisierung durch Kohlenmonoxid zersetzen, was zu einer Verclusterung von Rhodium führen kann. Die Rhodiumcluster können unter Hydroformylierungsbedingungen nicht zu dem aktiven Katalysator umgesetzt werden. Darüber hinaus kann bei der Destillation eine teilweise Zersetzung der Liganden auftreten.

Eine potentiell schonende Abtrennung von Homogenkatalysatorsystemen bietet die Aufkonzentrierung von homogenkatalysatorhaltigen Prozessströmen mit Membranen in ein- oder mehrstufiger Verschaltung.

In EP 0 781 166 wird die Abtrennung von gelöstem Rhodium-Organophosphit-Komplexkatalysator sowie freiem Liganden aus einer nicht-wässrigen Hydroformylierungs-Reaktionsmischung an einer Membran von mindestens 90 Massen-% des Katalysators und des freien Liganden beschrieben. Als Membranpolymer werden Teflon, Polydimethylsiloxan (PDMS), Polyethylen, Polyisobutadien, Polystyren, Polymethylmethacrylat, Polyvinylchlorid, Cellulosediacetat, Polyvinylidenechlorid und Polyacrylnitril aufgeführt. Die Abtrennung der Hochsieder vom Katalysatorsystem wird nicht beschrieben.

In EP 1 232 008 wird die Abtrennung von Hochsiedern aus einem Katalysator-Rückführstrom mittels einer Membran (PDMS) beschrieben. Der Rückführstrom fällt bei der destillativen Aufarbeitung eines Austrags einer metallorganisch katalysierten Reaktion an. Dabei werden Edukte und die primären Produkte abdestilliert und als Sumpfprodukt verbleibt ein Hochsiedergemisch, in dem das Katalysatorsystem gelöst ist. Dieses wird wieder in den Reaktor zurückgeführt. Da im Prozess geringe Mengen an Hochsieder gebildet werden, muss um die Konzentration an Hochsieder konstant zu halten, ein Teil davon abgetrennt werden. In EP 1 232 008 erfolgt die Abtrennung der Hochsieder aus dem Sumpfprodukt unter Zusatz eines Verdünnungsmittels. Es wird so viel Verdünnungsmittel zugesetzt, dass der Hochsiederanteil in der Lösung, die der Membran zugeführt wird, kleiner als 50 Massen-% ist. Die Abtrennung der Hochsieder geschieht im Temperaturbereich von 10 bis 50 °C und im Druckbereich von 0,1 bis 10 MPa. Die Zugabe von Verdünnungsmittel ist nachteilig, weil die über die Membran geführte Stoffmenge vergrößert wird. Weiterhin wird ein Teil des zugesetzten Verdünnungsmittels mit den Hochsiedern abgetrennt, wodurch Kosten für das Verdünnungsmittel oder für die Rückgewinnung daraus anfallen.

In DE 10 2005 046250 wird ein Verfahren zur Abtrennung eines metallorganischen Katalysatorsystems beschrieben. Dabei wird in einem ersten Schritt der organische Reaktionsaustrag in ein Retentat mit dem größeren Teil des Katalysatorsystems und in ein Permeat getrennt, das aus Edukten, primären Produkten, Hochsiedern und Katalysatorsystem besteht. Das Retentat wird direkt in den Reaktor zurückgeführt. Das Permeat wird destillativ in ein Kopfprodukt, das hauptsächlich Edukte und primäre Reaktionsprodukte enthält, und in ein Sumpfprodukt mit dem Katalysatorsystem, gelöst in Hochsiedern, getrennt. In dieser Schrift wird als optionale Aufarbeitungsvariante dargelegt, aus dem Sumpfprodukt, bevor es in den Reaktor zurückgeführt wird, einen Teil der Hochsieder mittels einer Membran abzutrennen. Als prinzipiell einsetzbare Membranmaterialien werden Polydimethylsiloxan (PDMS), Polyimid (PI), Polyamidimid (PAI), Acrylonitril/Glycidylmethacrylat (PANGMA), Polyamid (PA), Polyetheretherketon (PEEK), Polymere mit intrinsischer Mikroporosität (PIM) sowie hydrophobierte keramische Membranen genannt. Für die Hochsiederausschleusung mit Membranen werden allerdings keine Einzelheiten wie beispielsweise verwendbare Membrantypen genannt.

DE 10 2005 060784A1 wird ein Verfahren zur Rückgewinnung eines an einem Metallkomplexkatalysator (> 200 Dalton) angereicherten Stromes beschrieben. Der Reaktoraustrag wird destillativ in einen niedrig siedenden Strom und ein höher siedenden, den Katalysator enthaltenden, Sumpfstrom getrennt. Der Sumpfstrom wird mit einer Membran in einen Permeatstrom und einen Katalysator angereicherten Retentatstrom getrennt, welche vollständig oder teilweise der Reaktion zurück geführt wird. Es werden nur Keramikmembranen mit einer Trenngrenze (MWCO) von über 500 Dalton angegeben sowie Polymermembranen mit Trenngrenzen von über 10000 Dalton. Über die Aktivität als wesentliches Maß für die Qualität des zurückgehaltenen Katalysatorsystems wird keine Aussage gemacht.

Der beispielhaft angegebene Katalysator besitzt ein Molekulargewicht von ca. 12000 Dalton und liegt damit über eine Größenordnung über den üblichen technisch eingesetzten Katalysatoren. Eine Übertragung auf technisch relevante und von Molekulargewicht kleinere Katalysatorsysteme, ist mit der Darlegung aus DE 10 2005 060784A1 nicht möglich.

Zudem wird nicht angegeben, auf welchen Rückhalt sich die Trenngrenze bezieht und in welchem Stoffsystem die Trenngrenze ermittelt wurde. Die Angaben beziehen sich üblicherweise je nach Hersteller auf einen Rückhalt von 90 oder 95 %. Die Trenngrenze dient nicht als absolute Grenze sondern, als qualitative Hilfe für die Auswahl einer Membran für ein konkretes Trennproblem (siehe Melin, Rautenbach:Membranverfahren. 2. Auflage 2004 Springer- Verlag Berlin, Heidelberg.). Es ist daher fraglich, ob die angegebenen Trenngrenzen für den Metallkomplexkatalysator gelten. Die Notwendigkeit des Ligandrückhalts ist nicht erwähnt.

Die aufgelisteten Membranen sind ausschließlich poröse Membranen deren Trenngrenzen, wie weiter unten gezeigt wird, für das verfahrensgemäße Katalysatorsystem nicht geeignet sind. Zu den aufgelisteten Membranen werden neben diversen Keramikmembranen nur Polytetrafluorethylen, Polyvinylidenfluorid (PVDF), Polysulfon, Polyethersulfon, Polydimethylsiloxan (PDMS), Poletherketon, Polyamid und Polyimid als mögliche Membranmaterialien genannt.

In US 2006/0246273A1 wird ein neuartiges Polymer mit intrinsischer Mikroporosität (PIM) dargelegt. Aufgrund der starren Spirobindung verfügt dieses Polymer oder darauf basierende Polymermischungen über ein großes freies Volumen innerhalb der Polymermatrix. Das Polymer ist potentiell einsetzbar für die Trennung oder Anreicherung von Gas-, Dampf- oder Flüssiggemischen. Die Verwendung des Polymers ist aufgrund des hohen freien Volumens insbesondere für die Gasseparation als Membranwerkstoff geeignet. Fritsch et al. (J.Mem.Sci. 251, 263-269 (2005)) erreichen verhältnismäßig hohe Permeabilitäten.

Neben der Gasseparation sind auf PIM basierende Membranen potentiell auch einsetzbar zur Trennung chiraler Moleküle wie Aminosäuren, zur Trennung von Organika z. B. Alkohol aus wässrigen Systemen, zur Isomerentrennung, in Pharmazie und Biotechnologie zur Trennung von Proteinen oder anderer thermisch instabiler Komponenten, in Fermentern und Bioreaktoren zur Begasung und Biomassenabtrennung sowie zur Entfernung von Mikroorganismen aus Luft und Wasser.

Weitere potentielle Einsatzmöglichkeiten sind die Wasserreinigung, die Detektion oder Entfernung von Spurenkomponenten oder Metallsalzen aus Luft oder Wasser, die Trennung von Flüssigmischungen mit Pervaporation (z. B. bei der Ethanolproduktion) sowie die Gas-/Dampfseparation (z. B. Trennung organischer Dämpfe von Gas) und die Flüssig-Flüssig Trennung organischer Komponenten oder auch die Ausbeuteverbesserung bei Gleichgewichtsreaktionen durch selektive Produktausschleusung. Die Abtrennung von gelösten Feststoffen wie z. B. Homogenkatalysatoren ist nicht beschrieben. Auch sind die angegebenen Membrandicken von 10 bis 500 µm für die gewünschte Trennaufgabe zu dick.

In WO 2005/113121 A1 wird die Herstellung von Kompositmembranen mit einer dünnen Schicht aus einem mikroporösen Material mit hoher intrinsischer Mikroporosität beschrieben. Neben der Membranherstellung sind auch mögliche Anwendungen der Membranen genannt, wie die Fluidtrennung und die Trennung niedermolekularer Feststoffe von Fluiden. Erwähnt wird auch die Trennung von Wasserstoff und Kohlenwasserstoffen, N₂ oder CO, die Trennung von CO₂, H₂O und H₂S aus Erdgas, die Trennung von Stickstoff und Sauerstoff, die Trennung von VOCs (Volatile Organic Compounds) und anderen niederen Kohlenwasserstoffen von Luft und anderen Gasen, die Trennung von Spuren organischer Komponenten aus wässrigen Strömen sowie die Trennung von niedermolekularen Komponenten und Oligomeren von Fluiden und im Speziellen von Lösemitteln.

Nachteilig an den aus dem Stand der Technik bekannten Verfahren ist, dass die Verfahren entweder das Katalysatorsystem nicht ausreichend schonend und damit aktivitätserhaltend abtrennen können und/oder die aktiven Katalysatorsysteme nicht ausreichend zurückgehalten werden können. Insbesondere bei der Rückhaltung eines aktiven Katalysatorsystems von weniger als 1500 g/mol, welches eine wesentlich geringere Molmasse besitzt als die verclusterte Katalysatorspezies, besteht die Schwierigkeit mit einer Membran Komponenten mit einer Molmassendifferenz von wenigen 100 g/mol voneinander zu trennen. Gleichzeitig muss die Membran für die organischen Komponenten ausreichend permeabel sein, um einen wirtschaftlichen Prozess zu ermöglichen.

Da für die schonende Anreicherung eines Katalysatorsystems aus Prozessströmen der Hydroformylierung höherer Olefine (C4 und höher), bestehend aus dem Metallkomplexkatalysators und dessen Cluster sowie dem freien Liganden, keine befriedigende Lösung bekannt ist, bestand die Aufgabe, ein Verfahren zu entwickeln, dass den Katalysatorsystem enthaltenden Prozessstrom (z. B. Reaktoraustrag oder Sumpfprodukt) in einen Katalysatorsystem angereicherten Strom und einen Katalysatorsystem abgereicherten Strom auftrennt und einen großen Rückhaltegrad für das Katalysatorsystem, insbesondere für die Metallkomponente aufweist.

Die technische Aufgabe der Erfindung ist es daher, ein Verfahren zur Anreichung eines Homogenkatalysators zur Verfügung zu stellen, bei dem das Katalysatorsystem unter Erhaltung seiner Aktivität angereichert bzw. abgetrennt werden kann, wobei das Verfahren einen großen Rückhaltegrad für das Katalysatorsystem aufweisen muss, keine Verdünnungsmittel für die Rückgewinnung notwendig sind und auch keine Verclusterung des Metallanteils des Katalysators oder Zersetzung des Katalysatorkomplexes zu beobachten ist.

Diese technische Aufgabe wird gelöst durch ein Verfahren zur Anreicherung eines Homogenkatalysators aus einem Prozessstrom, der diesen Homogenkatalysator als Bestandteil enthält, wobei der Prozessstrom über mindestens eine Membran geführt wird und wobei die Membranen ganz oder teilweise aus einem Polymer besteht, das planare Polymereinheiten aufweist, die über einen starren Linker miteinander verbunden sind, wobei der Linker in sich verdreht ist, so dass mindestens ein planare Polymereinheit über den Linker in einer nicht coplanaren Anordnung mit mindestens einer zweiten planaren Polymereinheit verbunden ist.

Diese Polymere werden nachfolgend als Polymere mit intrinsischer Mikroporosität (PIM) bezeichnet.

Überraschender Weise wurde gefunden, dass sowohl aus einem organischen Reaktionsaustrag einer metallkatalysierten Reaktion, als auch aus einem aus dem Reaktionsaustrag erzeugten, mit Hochsiedern angereicherten Destillatsumpf, welcher Hochsieder, Metallkomplexe, dessen Cluster sowie freie Liganden enthält, mittels einer oder mehreren Membran(en) Hochsieder, Produkt und Edukte vom Katalysatorsystem abgetrennt werden können, wobei der Rückhalt für die Metallkomponente je Membran größer als 70 % ist und für den freien Liganden größer als 60 % ist, wenn die Trennung an Membranen, die Polymere mit intrinsischer Mikroporosität (PIM) enthalten, erfolgt.
Bevorzugt wird das Verfahren mit einer Trenngrenze von 400 bis 2000 g/mol im Temperaturbereich von 40 bis 150 °C und in einem Bereich des transmembranen Druckes (Differenzdruck über die Membran) von 5 bis 60 bar durchgeführt wird.

Auch bei Konzentrationen an Hochsiedern von über 50 Massen-% ist kein Zusatz eines Verdünnungsmittels notwendig, da auch bei hohen Konzentrationen an Hochsiedern der Permeatfluss und die Selektivität der Trennung für eine technische Realisierung ausreichend hoch ist. Überraschend ist, dass mit Membranen basierend auf PIM Katalysatorkomplexe, deren Molmassen in der gleichen Größenordnung wie die der Hochsieder liegen, besser als die Hochsieder zurückgehalten werden.

Hochsieder im Sinne der Erfindung sind Stoffe, die höher als die primären Hydroformylierungsprodukte (Aldehyde und/oder Alkohole mit einem C-Atom mehr als das eingesetzte Olefin) sieden und höhere Molmassen aufweisen und während der Hydroformylierung entstehen. Dazu gehören Aldolisierungsprodukte und Acetalisierungsprodukte sowie Ester, die durch Reaktion von Alkoholen und Säuren entstehen, wobei die Alkohole und Säuren durch Disproportionierung von Aldehyden gebildet werden. Hochsieder, die in Prozessströmen aus der Hydroformylierung vorhanden sind, besitzen im Allgemeinen Siedepunkte über 55 °C bei 0,1 MPa.

In einer bevorzugten Ausführungsform besitzen die Polymere innerhalb der Polymerstrukturen Spirobisindanbindungen, die als Linker fungieren.

In einer besonders bevorzugten Ausführungsform besitzen die Polymere innerhalb der 15 Polymerstrukturen substituierte oder unsubstituierte Spirobis[indan]substrukturen, die als Linker fungieren.

Spirobisindanbindungen im Sinne der Erfindung sind 1,1'-spirobis[indan], 1,2'-spirobis[indan] und 2,2'-spirobis[indan]; deren Strukturen lassen sich wie folgt darstellen:

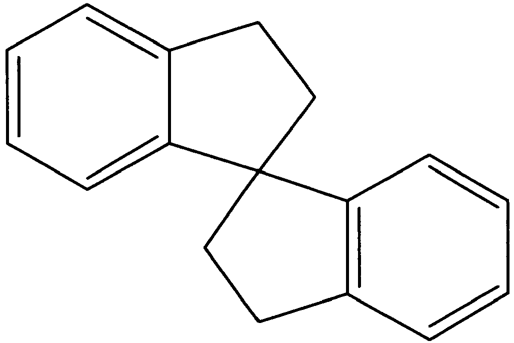

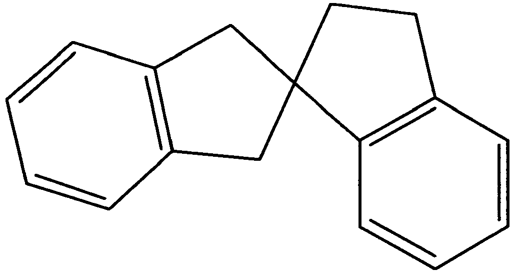

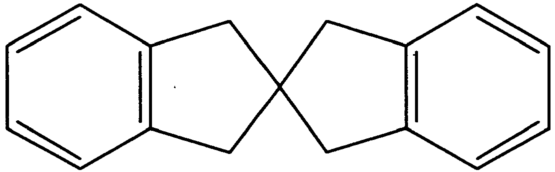

Die Spirobisindanbindung ist keine chemische Bindung im engen Sinne, sondern vielmehr eine zwischen den Polymeren angeordnete Substruktur.

Eine ganz besonders bevorzugte Spirobisindanbindung ist die Substruktur aus 3,3,3',3'-tetramethyl-1,1'spirobis[indan]:

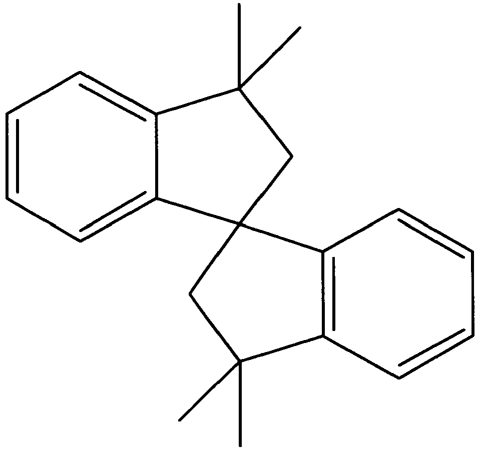

Als trennaktiver Membranwerkstoff sind besonders Polymere bevorzugt, die sich wiederholende Einheiten aus einer oder mehrer der nachstehenden Formel aufweisen, wobei n die Anzahl der Einheiten wiedergibt und vorzugsweise zwischen 10 und 100000 liegt.

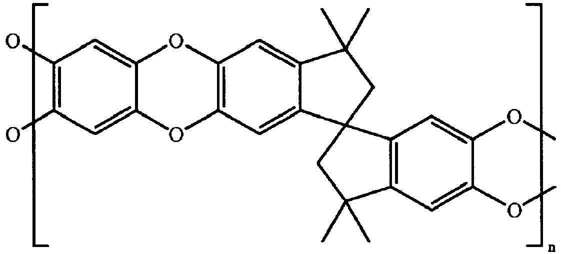

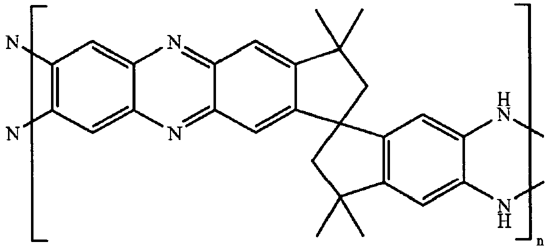

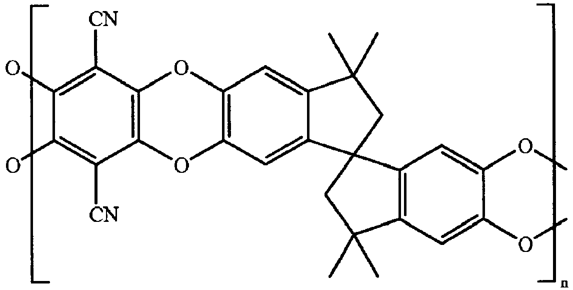

Die vorstehenden Polymere werden nachfolgend als PIM (Polymere mit intrinsischer Mikroporosität) bezeichnet.

Unter Anreicherung im Sinne der Erfindung wird nicht nur die Anreicherung des Homogenkatalysators verstanden, sondern auch die vollständige Abtrennung des Homogenkatalysators aus dem Prozessstrom.

Vorteilhaft ist, dass auf PIM basierte Membranen im Vergleich zu bisherigen Membranen einen besonders hohen Rückhalt von in organischen Lösemitteln gelösten Feststoffen wie Homogenkatalysatoren und deren Liganden aufweisen und gleichzeitig einen hohen Permeatfluss besitzen.

Ergänzend kann eine besonders schonende Abtrennung für das Katalysatorsystem erreicht werden, wenn das Katalysatorsystem möglichst in Gegenwart von Kohlenmonoxid und Wasserstoff aufgearbeitet wird, da hierdurch der Verclusterungsprozess zusätzlich vermieden werden kann. Eine Überlagerung mit Kohlenmonoxid als auch mit Wasserstoff ist insbesondere bei der Membranabtrennung möglich.

Die Synthesegasmenge sollte während der Abtrennung mindestens in der zur Bildung der aktiven Katalysatorspezies benötigten stöchiometrischen Menge überlagert sein, um die Bildung inaktiver oder sogar irreversibel ausfallender Katalysatorspezies verhindern zu können.

### Membranverschaltung

Das erfindungsgemäße Verfahren kann unter Einsatz von einer, zwei oder mehren Membran(en) oder unter Einsatz von einem, zwei oder mehreren Membrantrennschritten durchgeführt werden. Je nach Trennleistung der Membran und gewünschtem Rückhalt, kann, durch die Hintereinanderschaltung mehrerer Membrantrennschritte der gewünschte Rückhalt erzielt werden. In dem erfindungsgemäßen Verfahren können insbesondere zwei oder mehrere Membrantrennschritte durchgeführt werden. Die Membrantrennschritte können direkt hintereinander durchgeführt werden. Die Hintereinanderschaltung kann in der Weise erfolgen, dass entweder das Retentat oder das Permeat, vorzugsweise das Permeat eines ersten Membrantrennschritts als Zuführstrom in einen weiteren Membrantrennschritt geleitet wird. Die auf den ersten erfindungsgemäßen Membrantrennschritt gegebenenfalls folgenden Membrantrennschritte können ebenfalls unter ähnlichen Bedingungen wie der erste durchgeführt werden. In einem Membrantrennschritt können eine Membran oder mehrere Membrane eingesetzt werden. Vorzugsweise werden in einem Membrantrennschritt zwei oder mehrere Membrane eingesetzt.

Bei einem mehrstufigen Membrantrennverfahren kann es vorteilhaft sein in den Membrantrennschritten unterschiedliche Membranen einzusetzen. So wird bevorzugt in einem, einem Membrantrennschritt vorgeschalteten Membrantrennschritt, eine besser permeable Membran eingesetzt. Die dabei auftretende Klassierung führt zu einer besseren Permeabilität in dem nachgeschalteten Membrantrennschritt.

Im erfindungsgemäßen Verfahren ist die obere Temperaturgrenze bei den Membrantrennschritten durch die Stabilität der eingesetzten Membrane und die Stabilität des Katalysatorsystems vorgegeben. Die untere Temperaturgrenze ist abhängig von der Viskosität der zu trennenden Lösung und die temperaturabhängige Löslichkeit des Katalysatorsystems darin. Bevorzugt wird in dem erfindungsgemäßen Verfahren ein Membrantrennschritt, insbesondere der erste Membrantrennschritt, bei einer Temperatur von 40 bis 150 °C, besonders bevorzugt bei einer Temperatur von 60 bis 90 °C durchgeführt. Werden in dem erfindungsgemäßen Verfahren zur Abtrennung von Katalysator aus Hydroformylierungs-Reaktionsgemischen solche eingesetzt, die bei der Hydroformylierung von C₁₂-Olefinen erhalten werden, so wird der Membranschritt, insbesondere der erste Membranschritt vorzugsweise bei einer Temperatur von 60 bis 90 °C durchgeführt. Werden in dem erfindungsgemäßen Verfahren zur Abtrennung von Katalysator aus Hydroformylierungs-Reaktionsgemischen solche eingesetzt, die bei der Hydroformylierung von C₈-Olefinen erhalten werden, so wird der Membranschritt, insbesondere der erste Membranschritt vorzugsweise bei einer Temperatur von 40 bis 80 °C durchgeführt. Das Durchführen des erfindungsgemäßen Verfahrens bei den bevorzugten Temperaturen kann zum einen einen höheren Fluss durch die Membran bewirken. Zum anderen wird durch ein Einhalten der genannten bevorzugten Temperaturbereiche eine Zersetzung des Katalysators vermieden, die sonst zu Verlusten an aktivem Katalysator und zu einer Ablagerung von Zersetzungsprodukten des Katalysators auf der Membran führen kann. Durch eine Ablagerung kann der Mengenstrom durch die Membran gemindert werden. Im Extremfall kann der Mengenfluss durch Verblocken ganz zum Erliegen kommen.

Der transmembrane Druck (Druck an der Membran zwischen Retentat- und Permeatseite), bei dem das erfindungsgemäße Verfahren bevorzugt durchgeführt wird, beträgt vorzugsweise 5 bis 60 bar, besonders bevorzugt 10 bis 30 bar.

In dem erfindungsgemäßen Verfahren können PIM-Membranen eingesetzt werden, die auf Grund von ihren chemischen oder physikalischen Eigenschaften geeignet sind, Metall-Komplexkatalysatoren, insbesondere phosphororganische MetallKomplexkatalysator, in einem Maße von mindestens 60 %, insbesondere von über 80 % zurückzuhalten und eine Trenngrenze von 400 bis 2000 g/mol aufweisen. Eine weitere Voraussetzung für die Verwendbarkeit der Membran besteht darin, dass die Membran stabil gegenüber allen in der Reaktionsmischung vorhandenen Verbindungen, insbesondere gegenüber den Lösemitteln sein muss.

### PIM

Unter der Bezeichnung mikroporös werden auch Materialien verstanden, die als nanoporös bezeichnet werden könnten. Es existieren bisher zwei Klassen von intrinsisch mikroporösen Materialien. Zeolithe und amorphe Aktivkohle. Aufgrund der fehlenden Löslichkeit in organischen Lösemitteln können aus diesen Materialien keine Filme und damit auch keine Membranen hergestellt werden. Anders ist dies bei Polymeren.

Unter PIM (Polymer mit intrinsischer Mikroporosität) wird eine kürzlich neuentwickelte Polymerklasse bezeichnet, welche sich bevorzugt durch eine starre, innerhalb der Polymerstruktur eine Verdrehung bewirkende, Spirobisindanbindung auszeichnet. Die zufällige Verteilung der Spirobindung verhindert das Verdichten des Polymers. Die starre Verdrehung (der planaren Polymerketten) an der Spirobisindanbindung führt zu einem hohen Anteil an freien, untereinander verbundenen freien Volumen. Die Mikroporosität wird als intrinsisch bezeichnet, da sie unabhängig von thermischer Vorbehandlung und der Polymerverarbeitung auftritt.
Ein Beispiel für ein Polymer mit intrinsischer Mikroporosität ist das sogenannte PIM-1. Unter PIM-1 wird eines der ersten, in dieser Polymerklasse, synthetisierten Polymere verstanden. Der CA Index Name ist 1,4 benzenedicarbonitrile, 2,3,5,6-tetrafluoro- polymer with 2,2',3,3'-tetrahydro-3,3,3',3'-tetramethyl-1,1'-spirobi[1H-indene]-5,5',6,6'-tetrol mit der CA Nummer 676450-48-9. PIM-1 erhält man aus der Reaktion von 5,5',6,6'-Tetrahydroxy-3,3,3',3'-tetramethyl-1,1-spirobisindan mit 2,3,5,6-Tetrafluorophthalonitril :

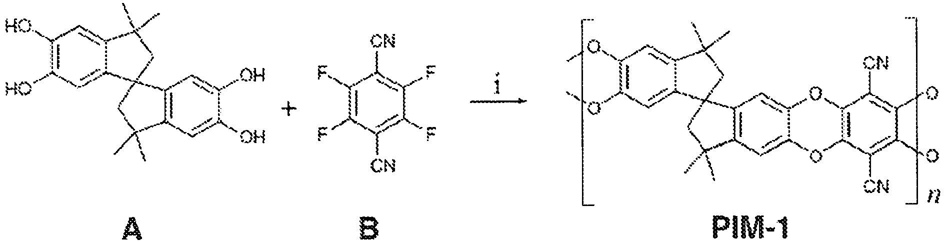

Weitergehende Beispiele für Polymere mit intrinsischer Mikroporosität sind in US2006/0246273A1 und in W02005/113121 dargelegt.

Weitere Beispiele an Comonomeren, die sich zur Modifizierung von PIM-1 eignen, sind:

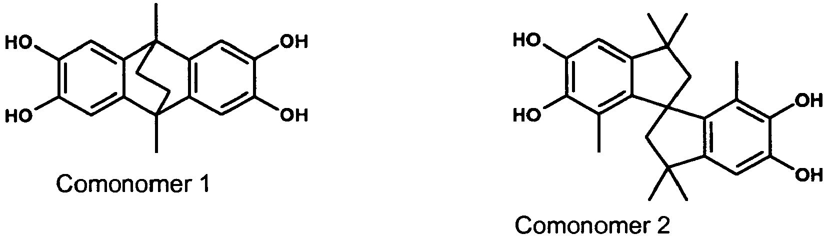

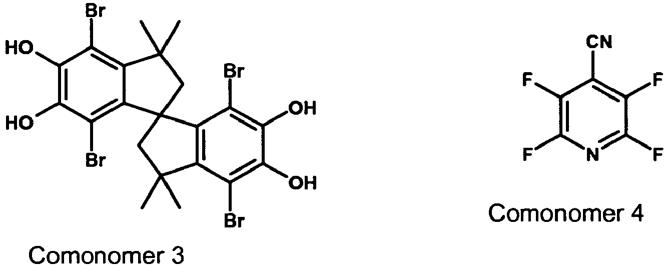

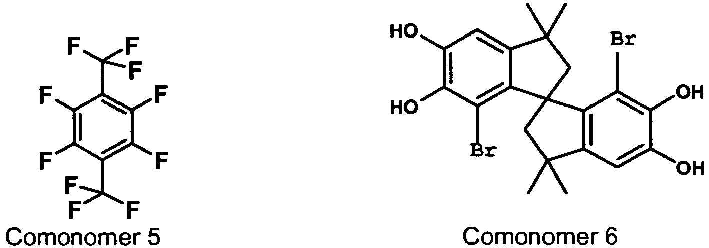

Das Comonomer 3 wird dabei durch Bromierung von Spirobisindan erzeugt.

Weitere Beispiele für Ausgangssubstanzen sind 9,9'-Spirobifluorene, modifiziert als 2,2'-Dicarboxy-9,9'-spiroflourene oder 2,2'-Diamino-9,9'-spirobifluorene, welche sich zur Herstellung von intrinsisch mikroporösen Polyamiden und Polyimiden eignen.

Neben den oben genannten Polymeren mit intrinsischer Mikroporosität können die Membrane weitere Materialien aufweisen. Insbesondere können die Membrane Stütz- oder Trägermaterialien aufweisen, auf die die PIM als dünne, trennaktive Schicht aufgebracht ist. Bei solchen Verbundmembranen liegt neben der eigentlichen Membran noch ein Stützmaterial vor. Eine Auswahl von Stützmaterialien beschreibt EP 0 781 166, auf welches explizit verwiesen wird. Weiterhin können in der erfindungsgemäß einsetzbaren Membran Verstärkungsmaterialien vorhanden sein, wie z. B. Partikel von anorganischen Oxiden oder anorganische Fasern, wie z. B. Keramik- oder Glasfasern, die die Stabilität der Membrane insbesondere gegenüber Druckschwankungen oder hohen Druckdifferenzen erhöhen. Die Dicke der Trennschicht der Membran für das erfindungsgemäße Verfahren beträgt vorzugsweise 10 - 1000 nm, besonders bevorzugt 100 - 700 nm.

### Technische Module

Die Membranen werden in dem erfindungsgemäßen Verfahren vorzugsweise in Form von Membranmodulen eingesetzt. In diesen Modulen werden die Membranen so angeordnet, dass die Retentatseite der Membran derart überströmt werden kann, dass die Konzentrationspolarisation der abgetrennten Komponenten, hier Katalysator-Ligandsystem, entgegengewirkt und außerdem die nötige treibende Kraft (Druck) aufgeprägt werden kann. Das Permeat wird im Permeatsammelraum auf der Permeatseite der Membran zusammengeführt und aus dem Modul abgeführt. Gängige Membranmodule weisen die Membrane in Form von Membranscheiben, Membrankissen oder Membrantaschen auf. Im erfindungsgemäßen Verfahren werden die Membrane vorzugsweise in Form von Membranmodulen eingesetzt, die Membranmodule mit offenkanaligen Kissenmodulsystemen, bei denen die Membranen zu Membrantaschen oder -kissen thermisch verschweißt oder verklebt sind, oder offenkanaligen (Wide-spacer) Wickelmodulen, bei denen die Membranen zu Membrantaschen oder Membrankissen verklebt oder verschweißt sind und mit Feed-spacern um ein Permeatsammelrohr aufgewickelt sind, aufweisen.

### Überströmung, Trennschritt

Um Ablagerungen auf der Membran zu vermeiden sind innerhalb der Membrantrennschritte gewisse Strömungsverhältnisse einzuhalten. Es hat sich gezeigt, dass die Ablagerungsgefährdung einer Strömung von deren Turbulenz und damit von ihrer Reynolds-Zahl abhängig ist. So ist unabhängig von der Bauart des Membranmoduls darauf zu achten, dass die Reynoldszahl zwischen 55 und 13500, bevorzugt zwischen 100 und 3500 und ganz besonders bevorzugt zwischen 170 und 900 liegt. Die kinematische Viskosität sollte dabei kleiner als 10 mPas und bevorzugt bei 1 mPas liegen. Bei diesen Strömungsverhältnissen werden Ablagerungen vermieden.

Zur Umsetzung dieser Strömungsverhältnisse wird bei der Verwendung von Wickelmembranen mit einer Rohrlänge von 1 m und einem Druckverlust von 1.5 bar und einer kinematischen Viskosität des Mediums von 1 mPas zur Vermeidung von Ablagerungen auf der Membran das Verfahren vorzugsweise so durchgeführt, dass der Membrantrennschritt, insbesondere der erste Membrantrennschritt mit einer Überströmgeschwindigkeit an der Membran von 0,1 bis 15 m/sec., bevorzugt 0,2 bis 4 m/sec, vorzugsweise von 0,3 bis 1 m/sec vorliegt.

Vorzugsweise wird das erfindungsgemäße Verfahren derart betrieben, dass die zu trennende Lösung als Zustrom auf die Membran gefahren und der Retentatstrom teilweise auf die Membran zurückgeführt wird. Dabei wird der Teilstrom, der auf die Membran zurückgeführt wird, zuvor mit der zu trennenden Lösung vereinigt. Der Teil des Retentatstroms, der nicht auf die Membran zurückgeführt wird, wird entweder als Zufuhrstrom für eine oder mehrere nachfolgende Abtrennstufen verwendet oder aber in die Reaktion zurückgeführt.

Wird dem Membrantrennschritt ein Strom mit geringem Anteil an Hochsiedern und hohem Anteil an primären Produkten, wie dies bei einem Reaktoraustrag ohne vorherige Einengung von Hochsiedern der Fall ist, zugeführt, beträgt das Volumenstrom Verhältnis von Permeatstrom zu Zufuhrstrom vom Reaktor (ohne rückgeführtem Retentat) 1 zu 1,1 bis 1 zu 5, bevorzugt von 1 zu 1,4 bis 1 zu 3 und besonders bevorzugt von 1 zu 1,6 bis 1 zu 2.

Wird im umgekehrten Fall dem Membrantrennschritt ein nach dem Reaktor, z.B. durch einen thermischen Trennschritt, ein gegenüber dem Reaktoraustrag an Hochsieder deutlich angereicherter Strom zugeführt, so beträgt das VolumenstromVerhältnis von Permeatstrom zu Zufuhrstrom vom Reaktor (ohne rückgeführtem Retentat) vorzugsweise 1 zu 5 bis 1 zu 20, bevorzugt von 1 zu 7,5 bis 1 zu 12,5 und besonders bevorzugt von 1 zu 9 bis 1 zu 11.

Es kann vorteilhaft sein, wenn der Volumenstrom, der über die Membran geführt wird, deutlich größer ist als der Volumenstrom des Permeatstroms, da auf diese einfache Weise eine hohe Überströmgeschwindigkeit an der Membran eingestellt werden kann. Vorzugsweise beträgt das Volumenstromverhältnis des auf die Membran, insbesondere auf die erste Membran des ersten Membrantrennschritts geführten Stroms (Zufluss vom Reaktor inklusive rückgeführtem Retentat) zu Permeatstrom von 10 bis 10000 zu 1, bevorzugt von 50 bis 5000 zu 1 und besonders bevorzugt von 200 bis 2000 zu 1. Es wird also vorzugsweise ein relativ hoher Volumenstrom im Kreis über die Membran geführt. Die Größe des Teils des Retentatstroms, der in die Reaktion zurückgeführt oder einer weiteren Abtrennung zugeführt wird, ergibt sich aus der Differenz von Zufuhrstrom (ohne rückgeführtem Retentat) und Permeatstrom.

Bei höheren Permeabilitäten kann es auch vorteilhaft sein, die Membranen in einer Tannenbaumstruktur zu verschalten.

Als Zuführstrom zur Membrantrennung kann der Reaktionsaustrag einer durch organische Metallkomplexe katalysierte Reaktion direkt oder ein daraus hergestelltes Konzentrat eingesetzt werden. Die Reaktionsausträge enthalten Edukte, primäre Produkte, Nebenprodukte wie beispielsweise Hochsieder, das Katalysatorsystem und gegebenenfalls ein Lösemittel. Wenn dieses Gemisch erfindungsgemäß aufgearbeitet wird, verbleibt das Katalysatorsystem, insbesondere der Metallkomplex, vorwiegend im Retentat. Mit dem Permeat, das in einer weiteren Trennstufe aufgearbeitet wird, werden Edukte, Produkte und Hochsieder zusammen abgetrennt. In diesem Falle ist der Permeatstrom wesentlich größer als der Retentatstrom, der nicht auf die Membran zurückgeführt wird. Dies bedingt eine große Membranfläche und einen nicht optimalen Rückhalt des Katalysatorsystems.

Vorzugsweise wird daher der größte Teil der Edukte und Produkte vorher durch einen thermischen Trennschritt abgetrennt. Ein solcher thermischer Trennschritt kann z. B. durch eine oder mehrere thermische Trennapparaturen, wie z. B. Dünnschichtverdampfer, Fallfilmverdampfer, Flashverdampfer oder Destillationskolonnen, realisiert werden. Als Kopfprodukt werden dabei im Wesentlichen Edukte und primäre Produkte abgetrennt. Als Sumpfprodukt fällt ein Gemisch mit Hochsieder und dem Katalysatorsystem an. Ist das Reaktionsgemisch ein Hydroformylierungsgemisch, enthält das Kopfprodukt üblicherweise das Hydroformylierungsprodukt, wie z. B. Aldehyd und/oder Alkohol sowie eventuell nicht umgesetzte Kohlenwasserstoffe, wie z. B. Olefine oder Aliphaten und gegebenenfalls bei der Hydroformylierung eingesetztes Lösemittel, welches eine Siedetemperatur im Bereich der Hydroformylierungsprodukte oder niedriger hat, welches einer weiteren Aufarbeitung zugeführt werden kann. Als Sumpfprodukt des thermischen Trennschrittes wird ein Gemisch erhalten, welches den Komplexkatalysator und/oder freien Liganden, gegebenenfalls ein höher als das Hydroformylierungsprodukt siedendes Lösemittel sowie während der Hydroformylierung entstandene Hochsieder enthält.

Der Anteil der Hochsieder im Sumpfprodukt liegt je nach Reaktionsbedingungen und Einsatzprodukt zwischen 50 und 98 Massen-%, insbesondere zwischen 60 und 90 Massen-%. Diese Lösungen können ohne Zusatz eines Verdünnungsmittels der Membrantrennung zugeführt werden.

Optional kann vor dem thermischen Trennschritt das Reaktionsgemisch mittels einer Membran in einen Strom mit höherer Katalysatorkonzentration und einen Strom mit niedrigerer Katalysatorkonzentration getrennt werden. Der Strom mit der höheren Katalysatorkonzentration wird in den Reaktor zurückgeführt. Der Strom mit der geringeren Katalysatorkonzentration wird dem thermischen Trennschritt zugeführt. Ein Verfahren dieser Art wird beispielsweise in DE 10 2005 046250.2 beschrieben.

Mit Hilfe der vorliegenden Erfindung können Gemische aufgetrennt werden, die bei Reaktionen unter Verwendung von homogen gelösten Metallkatalysatoren entstehen.

Als Beispiele für solche Reaktionen seien Hydrierungen, Hydroformylierungen, Metathesen, Hydrocyanierungen und Hydrocarboxyalkylierungen von Olefinen genannt. Bevorzugt werden Hydroformylierungsgemische, die RhodiumKomplexkatalysatoren enthalten, aufgearbeitet.

Die Hydroformylierungs-Reaktionsgemische können aus Verfahren zur Hydroformylierung von Olefinen, bevorzugt mit 2 bis 25 Kohlenstoffatomen, besonders bevorzugt 4 bis 16, ganz besonders bevorzugt von 6 bis 12 und insbesondere von 8, 9, 10, 11 oder 12 Kohlenstoffatomen, zu den entsprechenden Aldehyden stammen. Ganz besonders bevorzugt weist das Hydroformylierungs-Reaktionsgemisch als Hydroformylierungsprodukt einen Aldehyd auf, welcher ausgewählt ist aus Aldehyden mit 5 bis 17 Kohlenwasserstoffatomen, vorzugsweise 9 oder 13 Kohlenwasserstoffatomen, insbesondere Isononanal und Isotridecanal.

Die in dem Hydroformylierungs-Reaktionsgemisch vorhandenen Komplexkatalysatoren und/oder freien Organophosphor-Liganden können die aus dem Stand der Technik bekannten Verbindungen und Komplexe sein. Vorzugsweise weisen die Komplexkatalysatoren oder die freien Liganden solche Liganden auf, die ausgewählt sind aus den Phosphinen, Phosphiten, Phosphiniten, Phosphoniten. Die Liganden können dabei eine oder mehrere Phosphino-, Phosphito-, Phosphonito-oder Phosphinito-Gruppen aufweisen. Ebenso ist es möglich, dass die Liganden zwei oder mehr unterschiedliche Gruppen ausgewählt aus den Phosphino-, Phosphito-, Phosphonito- oder Phosphinito-Gruppen aufweisen. Insbesondere können die Liganden Bisphosphite, Bisphosphine, Bisphosphonite, Bisphosphinite, Phosphinphosphite, Phosphinphosphonite, Phosphinphosphinite, Phosphitphosphonite, Phosphitphosphinite oder Phosphonitphosphinite sein. Die Liganden des Komplexkatalysators und die freien Liganden können gleich oder unterschiedlich sein. Vorzugsweise sind die phosphororganischen Liganden des Komplexkatalysators und die freien Liganden gleich.

Beispiele für einsetzbare Komplexkatalysatoren bzw. Liganden und deren Herstellung und Verwendung bei der Hydroformylierung können z. B. EP 0 213 639, EP 0 214 622, EP 0 155 508, EP 0 781 166, EP 1209164, EP 1201675, DE 10114868, DE 10140083, DE 10140086, DE 10210918 oder WO 2003/078444 entnommen werden, auf welche ausdrücklich Bezug genommen wird.

Beispiele für bevorzugte Liganden sind:
Phosphine: Triphenylphosphin, Tris(p-tolyl)phosphin, Tris(m-tolyl)phosphin, Tris(o-tolyl)phosphin, Tris(p-methoxyphenyl)phosphin, Tris(p-dimethylamino-phenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin.

Phosphite: Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2,4-di-t-butylphenyl)phosphit, Tris(2-t-butyl-4-methoxyphenyl)phosphit, Tris(2-t-butyl-4-methylphenyl)phosphit, Tris(p-kresyl)phosphit.

Phosphonite: Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind.

Gängige Phosphinitliganden sind Diphenyl(phenoxy)phosphin und dessen Derivate Diphenyl(methoxy)phosphin und Diphenyl(ethoxy)phosphin.

Weiterhin können die Hydroformylierungsgemische ein Acyl- oder Heteroacylphosphit oder einen Acyl- oder Heteroacylphosphit-Gruppe aufweisenden Ligand als Organophosphor-Ligand aufweisen. Acylphosphite oder Acylphosphit-Gruppen aufweisende Liganden, deren Herstellung und deren Einsatz in der Hydroformylierung werden beispielsweise in DE 100 53 272 beschrieben. Heteroacylphosphite und Heteroacylphosphit-Gruppen aufweisende Liganden, deren Herstellung und deren Einsatz in der Hydroformylierung werden beispielsweise in DE 10 2004 013 514 beschrieben.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll.

Das erfindungsgemäße Verfahren zur Abtrennung von höher siedenden Komponenten von Homogenkatalysatoren oder Homogenkatalysatorsystemen, durch Abtrennung mit Membranen basierend auf einem Polymer mit intrinsischer Mikroporosität (PIM) wird im Folgenden beispielhaft für die rhodiumkomplexkatalysierte Hydroformylierung von C₄- bis C₁₆- Olefinen beschrieben.

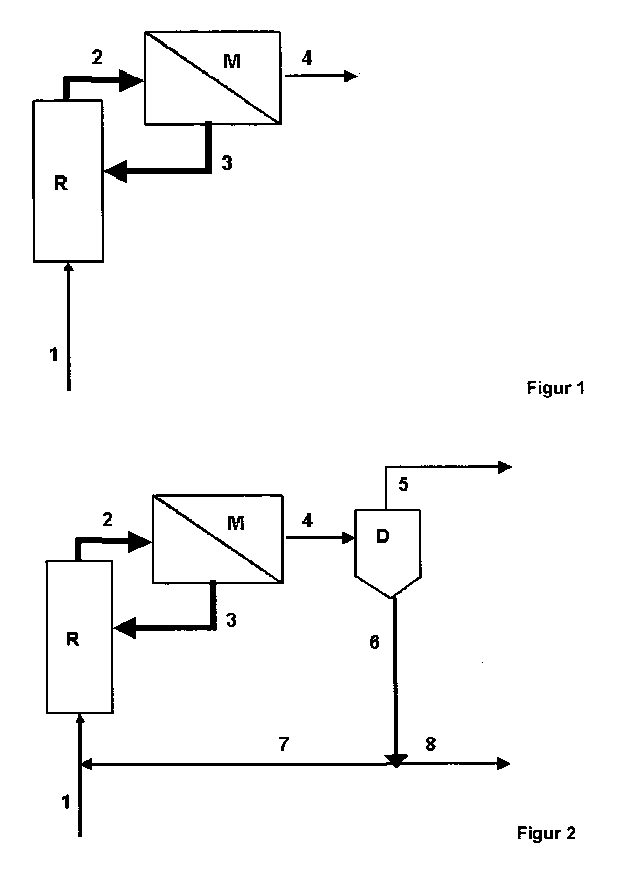

Nach Fig. 1 werden die Edukte (1) der Hydroformylierung, Olefin und Synthesegas, dem Reaktor (R) zugeführt. In Gegenwart des im Reaktor vorgelegten Katalysatorsystems findet die Oxierung des Olefins zu Aldehyd statt. Umgesetztes Edukt wie Aldehyd und Neben- und Folgeprodukte darunter Hochsieder wie Aldolkondensationsprodukte und nicht umgesetztes Edukt sowie das Katalysatorsystem werden als Reaktionsgemisch (2) aus dem Reaktor abgeführt und einem selektiven Membrantrennschritt (M) zugeführt. Dabei findet retentatseitig (3) eine Anreicherung und permeatseitig (4) eine Abreicherung des Katalysators oder des Katalysatorsystems statt.

Fig. 2 zeigt eine Erweiterung des Verfahrens aus Fig. 1, indem das Permeat (4) des Membrantrennschritts einem thermischen Trennschritt (D) zugeführt wird. In dem thermischen Trennschritt wird der Permeatstrom (4) in einen an höher siedende Komponenten, wie Nebenprodukte des Reaktionsteils sowie im Membrantrennschritt nicht zurückgehaltene Katalysatorkomponenten angereicherten Strom (6) und in einen an leichter siedenden, überwiegend Aldehyd enthaltenden Produktstrom (5) getrennt. Um eine Anreicherung an höher siedenden Nebenprodukten im Prozess zu vermeiden, kann Strom (6) oder ein Teilstrom (8) von Strom (6) aus dem Prozess ausgeschleust werden. Sofern es nicht erforderlich ist den gesamten Strom (6) auszuschleusen, kann es vorteilhaft sein den verbleibenden Strom (7) der Reaktion zurückzuführen, insbesondere, wenn Strom (7) noch Teile des Katalysatorsystems enthält.

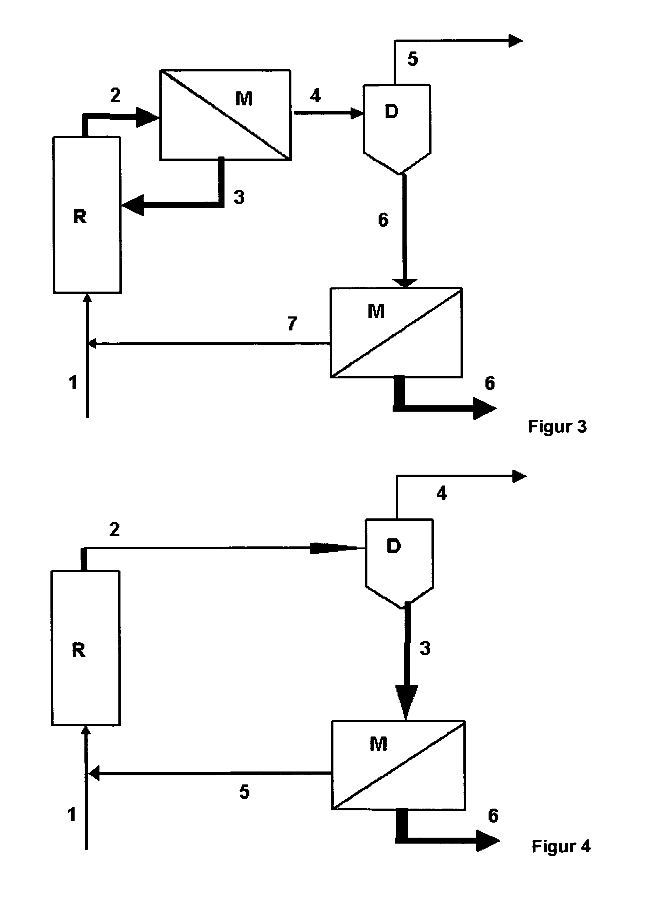

Je nach Werthaltigkeit des Strom (6) an Katalysatorkomponenten, kann es vorteilhaft sein Strom 6 durch einen zweiten Membrantrennschritt in einen gegenüber Strom (6) mit Katalysator angereicherten Strom (7) und einen entsprechend mit Katalysator abgereicherten Strom (8) zur Ausschleusung von hochsiedenden Komponenten aufzutrennen (siehe Fig. 3). Je nach Aktivität des in Strom (7) angereicherten Katalysators kann es vorteilhaft sein diesen Strom der Reaktion direkt oder indirekt zuzuführen.

Die Verschaltung nach Fig. 3 kann auch ohne den Membrantrennschritt nach dem Reaktionsaustrag vorteilhaft sein. Eine solche Verschaltung zeigt Fig. 4.

Ein weiterer Gegenstand der Erfindung ist ein Verfahren zur Herstellung von Tridecanal, das die folgenden Schritte umfasst:
a. Hydroformylierung von Tributen zu Tridecanal unter Verwendung eines homogenen Katalysatorsystems, bestehend aus Rhodium und einer Organophosphorverbindung,
b. destillative Auftrennung des Reaktionsaustrages in ein Destillat, das nicht umgesetzte Olefine und Aldehyde enthält und ein Sumpfprodukt das Hochsieder und das Katalysatorsystem enthält,
c. Durchführung des erfindungsgemäßen Verfahrens zur Anreicherung eines Homogenkatalysators, wobei die Hochsieder als Permeat und das Katalysatorsystem als Retentat voneinander getrennt werden,
d. Rückführung des Retentats mit dem angereicherten Katalysatorsystem in den Hydroformylierungsreaktor.

In einer bevorzugten Ausführungsform wird zur Herstellung des Tridecanals ein Tributen eingesetzt, das durch Oligomerisierung von linearen Butenen an Nickel enthaltenden Festkatalysatoren hergestellt wird.

In besonders bevorzugter Ausführungsform ist die Organophosphorverbindung Tris(2,4-ditertiärbutylphenyl)phosphit.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen. Nachfolgend wird die vorliegende Erfindung anhand von Beispielen näher erläutert. Alternative Ausführungsformen der vorliegenden Erfindung sind in analoger Weise erhältlich.

### Beispiele

### Erfindungsgemäßes Beispiel

Als Beispiel wurde die sich aus den vorherigen Verschaltungen für den Membrantrennschritt, aufgrund des hohen Hochsiederanteiles, anspruchsvollste Trennaufgabe gemäß Fig. 4 gewählt. Bei der Reaktion handelt es sich um eine Rhodium/Phosphit-katalysierte Hydrofomylierung eines Dodecengemisches (Tri-n-Buten). Der eingesetzte Rhodiumvorläufer ist Rhodiumacetylacetonatodicarbonyl und der eingesetzte Ligand ist Tris(2,4-di-tert-butyl-phenyl)phosphit. Die Rhodiumkonzentration betrug 10 mg/kg. Das Rhodium zu Ligandverhältnis betrug 20. Umgesetztes Edukt wie der Aldehyd (Isotridecanal) und Neben- und Folgeprodukte darunter Hochsieder wie Aldolkondensationsprodukte und nicht umgesetztes Edukt sowie das Katalysatorsystem werden einem thermischen Trennschritt zugeführt, dessen an Hochsieder und an Katalysator angereicherter Sumpf wird dem Membrantrennschritt zugeführt. Der Hochsiederanteil liegt bei über 50 %. Die Molmassendifferenz zwischen aktiver Katalysatorspezies und den Hochsiedern liegt unter 500 g/mol.

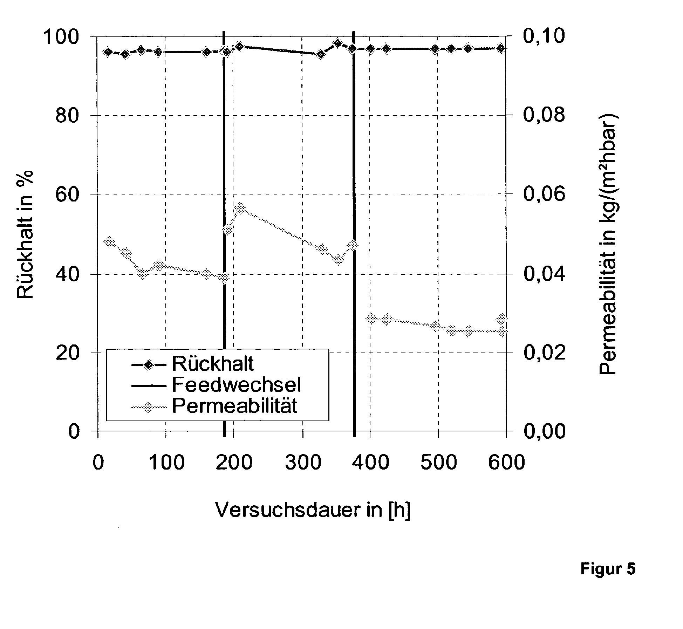

Beispielhaft konnte gezeigt werden, dass der Rhodiumrückhalt mit einer PIM-Membran im Hochsiederstrom bei über 95 % liegt (Fig. 5). Die Membranfiltration wurde dabei beispielhaft bei einem Transmembrandruck von 25 bar und einer Temperatur von 60 °C durchgeführt. Die feedseitige Überströmung betrug 1,7m/s, der Rohrdruckverlust lag unter 1 bar, die Reynoldszahl betrug etwa 1200. Dieser hohe Rückhalt konnte für eine Versuchsdauer von über 20 Tage nachgewiesen werden. Die dabei erreichten Permeatflüsse liegen zwischen 0,63 und 1,41 kg/(m²h). Die Rhodiumkonzentrationen im Feed des Membrantrennschritts liegen bei 50 bis 133 mg/kg.

### Vergleichsbeispiel

Versuche haben gezeigt, dass sich die Verfahrensvorschrift aus DE 10 2005 060784A1 nicht für die verfahrensgemäße Trennaufgabe anwenden lässt. Wesentlicher Grund ist die nicht ausreichende Trennschärfe der darin genannten Membranen und Membranmaterialien.

Tab. 1 zeigt die entsprechenden Ergebnisse für eine Auswahl von in DE 10 2005 060784A1 genannter Membrantypen. Bei dem Feed des Membrantrennschritts handelt es sich, wie bei dem verfahrensgemäßem Beispiel, um einen durch einen thermischen Trennschritt an Hochsieder angereicherten Reaktoraustragsstrom der Rhodium/Phosphit-katalysierten Hydrofomylierung von Dodecen. Entsprechend beinhaltet dieser Strom umgesetztes Edukt wie den Aldehyd (Isotridecanal) und Neben- und Folgeprodukte, darunter Hochsieder wie Aldolkondensationsprodukte und nicht umgesetztes Edukt sowie das Katalysatorsystem. Die Keramikmembranen von Inopor^{®} und Velterop^{®} weisen einen guten Permeatfluss auf, können aber das Katalysatorsystem bei weitem nicht ausreichend zurückhalten. Die Polymermembranen besitzen einen gegenüber den Keramikmembranen erhöhten, jedoch nicht ausreichenden Rückhalt für den Katalysator. Die Polyimidmembran Starmem 240^{®} weist zudem eine ungeeignet niedrige Permeabilität aus.

**Tab. 1 TMP = transmembraner Druck**

| **Herst./Membran** | **Material** | **Rh Feed** | **Rh Permeat** | **Rückhalt** | **Fluss** | **TMP** | **Temp.** |
|---|---|---|---|---|---|---|---|
| | | [mg/kg] | [mg/kg] | **[%]** | [kg/(h*m²)] | [bar] | [°C] |
| Inopor nano 0,9nm | Ti02 | 20 | 11 | **45,0** | 3,08 | 20 | 121 |
| Velterop 0,1 nm | Al2O3 | 77 | 62 | **19,5** | 11,42 | 15 | 150 |
| MET Starmem240 | PI | 120 | 62,5 | **47,9** | 0,29 | 30 | 90 |
| GMT oNF2 | PDMS | 120 | 27,5 | **77,1** | 1,74 | 25 | 60 |

Die Beispiele zeigen, dass mit dem erfindungsgemäßen Verfahren gegenüber den Verfahren des Standes der Technik eine erheblich bessere Anreicherung des Katalysatorsystems möglich ist. Damit kann das Verfahren aufgrund des höheren Rückhaltegrads für das Katalysatorsystem erheblich wirtschaftlicher durchgeführt werden. Dies trifft insbesondere zu bei Katalysatorsystemen, die wertvolle Metalle wie Rhodium enthalten.

## Patentansprüche

1. Verfahren zur Anreicherung eines Homogenkatalysators aus einem Prozessstrom, der diesen Homogenkatalysator als Bestandteil enthält, wobei der Prozessstrom über mindestens eine Membran geführt wird und wobei die Membran ganz oder teilweise aus einem Polymer mit intrinsischer Mikroporosität besteht, das planare Polymereinheiten aufweist, die über einen starren Linker miteinander verbunden sind, wobei der Linker in sich verdreht ist, so dass mindestens eine planare Polymereinheit über den Linker in einer nicht coplanaren Anordnung mit mindestens einer zweiten planaren Polymereinheit verbunden ist,
wobei das Polymer innerhalb der Polymerstruktur Spirobisindanbindungen aufweist, die als Linker fungieren,
**dadurch gekennzeichnet,**
**dass** der Prozessstrom Hochsieder und Homogenkatalysator aus einem homogen katalysierten organischen Reaktionsaustrag einer durch organische Metallkomplexe katalysierten Reaktion enthält, und der Hochsieder mit dem Permeat abgetrennt wird und das Katalysatorsystem im Retentat verbleibt,
wobei es sich bei dem Prozessstrom um einen aus dem Reaktionsaustrag erzeugten, mit Hochsiedern angereicherten Destillatsumpf handelt.

2. Verfahren nach Anspruch 1, **dadurch gekennzeichnet, dass** Polymer sich wiederholende Einheiten aus einem oder mehreren der nachstehenden Formeln aufweist, wobei n die Anzahl der Einheiten ist und vorzugsweise im Bereich von 10 bis 100000 liegt.

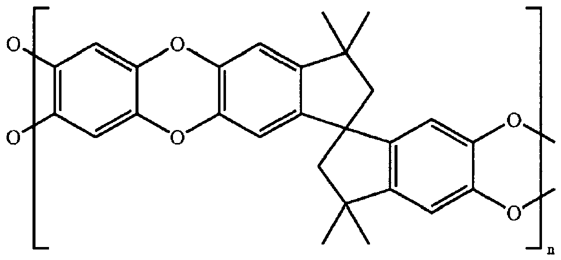

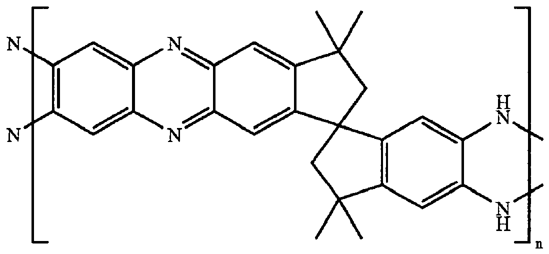

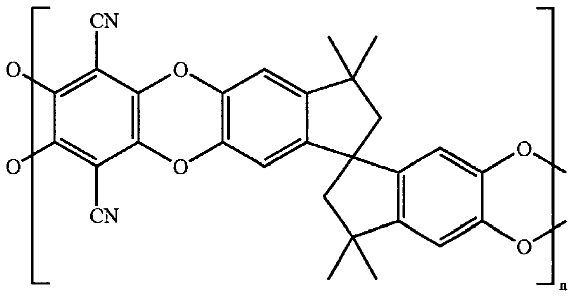

3. Verfahren nach Anspruch 2, **dadurch gekennzeichnet, dass** der Homogenkatalysator einen oder mehrere Metallkomplexe enthält.

4. Verfahren nach einem oder mehreren der vorhergehenden Ansprüche, **dadurch gekennzeichnet, dass** das Verfahren an Membranen mit einer Trenngrenze von 200 bis 2000 g/mol durchgeführt wird.

5. Verfahren nach einem oder mehreren der vorhergehenden Ansprüche, **dadurch gekennzeichnet, dass** das Verfahren bei einer Temperatur von 40 bis 150 °C durchgeführt wird.

6. Verfahren nach einem oder mehreren der vorhergehenden Ansprüche, **dadurch gekennzeichnet, dass** das Verfahren bei einem transmembranen Druck von 0,5 bis 6 MPa durchgeführt wird.

7. Verfahren nach einem oder mehreren der vorhergehenden Ansprüche, **dadurch gekennzeichnet, dass** das Verfahren in Gegenwart von Kohlenmonoxid und/oder Wasserstoff durchgeführt wird.

8. Verfahren nach einem oder mehreren der vorhergehenden Ansprüche, **dadurch gekennzeichnet, dass** das Verfahren unter Einsatz von 1 bis 3 Membranen durchgeführt wird.

9. Verfahren nach einem oder mehreren der vorhergehenden Ansprüche, **dadurch gekennzeichnet, dass** das Verfahren unter Einsatz von 1 bis 3 Membrantrennschritten durchgeführt wird.

10. Verfahren nach einem oder mehreren der vorhergehenden Ansprüche, **dadurch gekennzeichnet, dass** der Anteil der Hochsieder im Prozessstrom bei 50 bis 98 Massen-% liegt.

11. Verfahren nach einem oder mehreren der vorhergehenden Ansprüche, **dadurch gekennzeichnet, dass** das Verfahren ohne Zusatz eines Verdünnungsmittels durchgeführt wird.

12. Verfahren nach einem oder mehreren der vorhergehenden Ansprüche, **dadurch gekennzeichnet, dass** Homogenkatalysatoren aus Metallkomplexen, die mindestens ein Metall der 4., 5., 6., 7., 8., 9. oder 10. Gruppe des Periodensystems der Elemente und mindestens einen organischen Liganden aufweisen, von den Hochsiedern getrennt werden.

13. Verfahren nach Anspruch 12, **dadurch gekennzeichnet, dass** Metallkomplexkatalysatoren enthaltend Organophosphorverbindungen vom Hochsieder abgetrennt werden.

14. Verfahren nach Anspruch 13, **dadurch gekennzeichnet, dass** Metallkomplexkatalysatoren, die Rhodium und Organophosphorverbindungen enthalten, vom Hochsieder abgetrennt werden.

15. Verfahren nach Anspruch 14, **dadurch gekennzeichnet, dass** die Hochsieder aus einem Prozessstrom eines Hydroformylierungsgemisches abgetrennt werden, wobei das Hydroformylierungsgemisch Hochsieder, mindestens einen Komplexkatalysator, der Rhodium und eine Organophosphorverbindung enthält und Organophosphorverbindungen aufweist.

16. Verfahren nach einem oder mehreren der vorhergehenden Ansprüche, **dadurch gekennzeichnet, dass** die Trennschichtdicke der Membran 10 bis 1000 nm beträgt.

17. Verfahren zur Herstellung von Tridecanol, das folgende Schritte umfasst:
a. Hydroformylierung von Tributen zu Tridecanol unter Verwendung eines homogenen Katalysatorsystems, bestehend aus Rhodium und einer Organophosphorverbindung,
b. destillative Auftrennung des Reaktionsaustrages in ein Destillat, das nicht umgesetzte Olefine und Aldehyde enthält und ein Sumpfprodukt das Hochsieder und das Katalysatorsystem enthält,
c. Durchführung des Verfahrens gemäß den Ansprüchen 1 bis 16, wobei die Hochsieder als Permeat und das Katalysatorsystem als Retentat voneinander getrennt werden,
d. Rückführung des Retentats mit dem angereicherten Katalysatorsystem in den Hydroformylierungsreaktor.

18. Verfahren nach Anspruch 17, **dadurch gekennzeichnet, dass** als Organophosphorverbindung Tris(2,4-ditertiärbutlyphenyl)phosphit eingesetzt wird.

## Claims

1. Method of concentrating a homogeneous catalyst from a process stream containing this homogeneous catalyst as constituent, wherein the process stream is passed over at least one membrane and the membrane consists entirely or partially of a polymer having intrinsic microporosity and comprising planar polymer units which are connected to one another via a rigid linker, with the linker being intrinsically twisted so that at least one planar monomer unit is joined via the linker in a noncoplanar arrangement to at least one second planar polymer unit,
wherein the polymer has spirobisindane bonds which function as linkers within the polymer structure,
**characterized**
**in that** the process stream contains high boilers and homogeneous catalysts from a homogeneously catalyzed organic reaction output of a reaction catalyzed by organic metal complexes and the high boilers are separated off with the permeate and the catalyst system remains in the retentate, wherein the process stream is distillation bottoms which have been produced from the reaction output and are enriched in high boilers.

2. Method according to Claim 1, **characterized in that** the polymer comprises repeating units having one or more of the following formulae, where n is the number of units and is preferably in the range from 10 to 100 000.

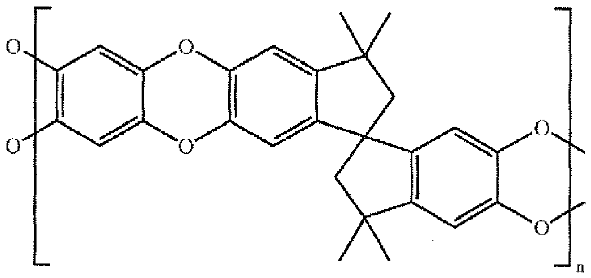

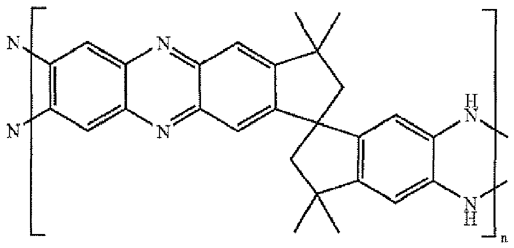

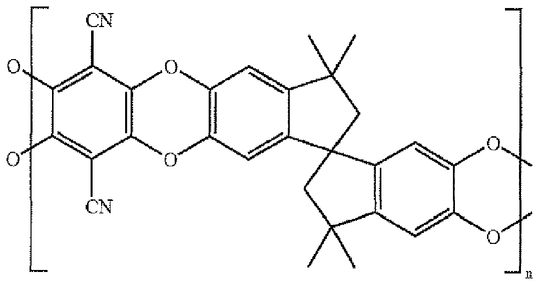

3. Method according to Claim 2, **characterized in that** the homogeneous catalyst contains one or more metal complexes.

4. Method according to one or more of the preceding claims, **characterized in that** the method is carried out over membranes having a separation limit of from 200 to 2000 g/mol.

5. Method according to one or more of the preceding claims, **characterized in that** the method is carried out at a temperature of from 40 to 150°C.

6. Method according to one or more of the preceding claims, **characterized in that** the method is carried out at a transmembrane pressure of from 0.5 to 6 MPa.

7. Method according to one or more of the preceding claims, wherein the method is carried out in the presence of carbon monoxide and/or hydrogen.

8. Method according to one or more of the preceding claims, **characterized in that** the method is carried out using from 1 to 3 membranes.

9. Method according to one or more of the preceding claims, **characterized in that** the method is carried out using from 1 to 3 membrane separation steps.

10. Method according to one or more of the preceding claims, **characterized in that** the proportion of high boilers in the process stream is from 50 to 98% by mass.

11. Method according to one or more of the preceding claims, **characterized in that** the method is carried out without addition of a diluent.

12. Method according to one or more of the preceding claims, **characterized in that** homogeneous catalysts composed of metal complexes comprising at least one metal of group 4, 5, 6, 7, 8, 9 or 10 of the Periodic Table of the Elements and at least one organic ligand are separated from the high boilers.

13. Method according to Claim 12, **characterized in that** metal complex catalysts containing organophosphorus compounds are separated off from the high boilers.

14. Method according to Claim 13, **characterized in that** metal complex catalysts containing rhodium and organophosphorus compounds are separated off from the high boilers.

15. Method according to Claim 14, **characterized in that** the high boilers are separated off from a process stream of a hydroformylation mixture, where the hydroformylation mixture comprises high boilers, at least one complex catalyst containing rhodium and an organophosphorus compound and organophosphorus compounds.

16. Method according to one or more of the preceding claims, **characterized in that** the separation layer thickness of the membrane is from 10 to 1000 nm.

17. Process for preparing tridecanol, which comprises the following steps:
a. hydroformylation of tributene to tridecanol using a homogeneous catalyst system comprising rhodium and an organophosphorus compound,
b. separation of the reaction output by distillation into a distillate containing unreacted olefins and aldehydes and a bottom product containing high boilers and the catalyst system,
c. carrying out the method according to any of Claims 1 to 16, where the high boilers as permeate and the catalyst system as retentate are separated from one another,
d. recirculation of the retentate with the concentrated catalyst system to the hydroformylation reactor.

18. Process according to Claim 17, **characterized in that** tris(2,4-di-tert-butylphenyl) phosphite is used as organophosphorus compound.

## Revendications

1. Procédé d'enrichissement d'un catalyseur homogène à partir d'un courant de procédé, qui contient ce catalyseur homogène en tant que constituant, le courant de procédé traversant au moins une membrane et la membrane étant constituée en totalité ou en partie d'un polymère à microporosité intrinsèque qui comprend des unités polymères planes, qui sont reliées les unes aux autres par un coupleur rigide, le coupleur étant tordu de manière à ce qu'au moins une unité polymère plane soit reliée par le coupleur selon un agencement non coplanaire avec au moins une deuxième unité polymère plane,
le polymère comprenant dans la structure polymère des liaisons spirobisindane qui servent de coupleur, **caractérisé en ce que**
le courant de procédé contient des composés de point d'ébullition élevé et un catalyseur homogène issus d'une sortie de réaction organique sous catalyse homogène d'une réaction catalysée par des complexes métalliques organiques, et le composé de point d'ébullition élevé est séparé avec le perméat et le système catalytique reste dans le rétentat,
le courant de procédé étant un fond de distillation enrichi en composés de point d'ébullition élevé formé à partir d'une sortie de réaction.

2. Procédé selon la revendication 1, **caractérisé en ce que** le polymère comprend des unités de répétition d'une ou de plusieurs des formules suivantes, n étant le nombre d'unités et se situant de préférence dans la plage allant de 10 à 100 000

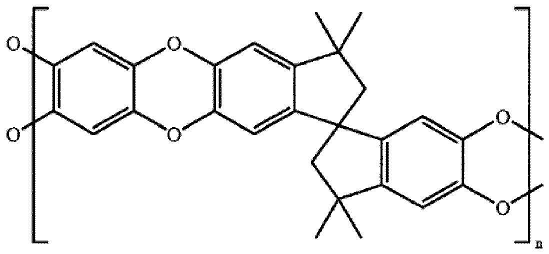

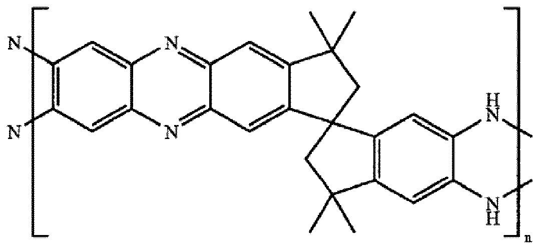

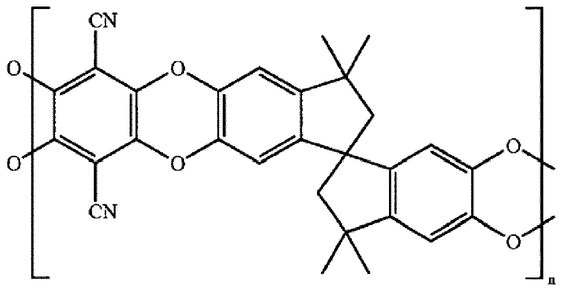

3. Procédé selon la revendication 2, **caractérisé en ce que** le catalyseur homogène contient un ou plusieurs complexes métalliques.

4. Procédé selon une ou plusieurs des revendications précédentes, **caractérisé en ce que** le procédé est réalisé sur des membranes présentant un seuil de séparation de 200 à 2 000 g/mol.

5. Procédé selon une ou plusieurs des revendications précédentes, **caractérisé en ce que** le procédé est réalisé à une température de 40 à 150 °C.

6. Procédé selon une ou plusieurs des revendications précédentes, **caractérisé en ce que** le procédé est réalisé à une pression transmembranaire de 0,5 à 6 MPa.

7. Procédé selon une ou plusieurs des revendications précédentes, **caractérisé en ce que** le procédé est réalisé en présence de monoxyde de carbone et/ou d'hydrogène.

8. Procédé selon une ou plusieurs des revendications précédentes, **caractérisé en ce que** le procédé est réalisé en utilisant 1 à 3 membranes.

9. Procédé selon une ou plusieurs des revendications précédentes, **caractérisé en ce que** le procédé est réalisé en utilisant 1 à 3 étapes de séparation sur membrane.

10. Procédé selon une ou plusieurs des revendications précédentes, **caractérisé en ce que** la proportion des composés de point d'ébullition élevé dans le courant de procédé est de 50 à 98 % en masse.

11. Procédé selon une ou plusieurs des revendications précédentes, **caractérisé en ce que** le procédé est réalisé sans ajout d'un diluant.

12. Procédé selon une ou plusieurs des revendications précédentes, **caractérisé en ce que** des catalyseurs homogènes de complexes métalliques comprenant au moins un métal du groupe 4, 5, 6, 7, 8, 9 ou 10 du tableau périodique des éléments et au moins un ligand organique sont séparés des composés de point d'ébullition élevé.

13. Procédé selon la revendication 12, **caractérisé en ce que** des catalyseurs de complexes métalliques contenant des composés d'organophosphore sont séparés du composé de point d'ébullition élevé.

14. Procédé selon la revendication 13, **caractérisé en ce que** des catalyseurs de complexes métalliques contenant du rhodium et des composés d'organophosphore sont séparés du composé de point d'ébullition élevé.

15. Procédé selon la revendication 14, **caractérisé en ce que** les composés de point d'ébullition élevé sont séparés d'un courant de procédé d'un mélange d'hydroformylation, le mélange d'hydroformylation comprenant des composés de point d'ébullition élevé, au moins un catalyseur complexe, qui contient du rhodium et un composé d'organophosphore, et des composés d'organophosphore.

16. Procédé selon une ou plusieurs des revendications précédentes, **caractérisé en ce que** l'épaisseur de la couche de séparation de la membrane est de 10 à 1 000 nm.

17. Procédé de fabrication de tridécanol, qui comprend les étapes suivantes :
a. l'hydroformylation de tributène en tridécanol en utilisant un système catalytique homogène constitué par du rhodium et un composé d'organophosphore,
b. la séparation par distillation de la sortie de réaction en un distillat, qui contient des oléfines non réagies et des aldéhydes, et un produit de fond, qui contient des composés de point d'ébullition élevé et le système catalytique,
c. la réalisation du procédé selon les revendications 1 à 16, les composés de point d'ébullition élevé étant séparés en tant que perméat et le système catalytique en tant que rétentat,
d. le recyclage du rétentat contenant le système catalytique enrichi dans le réacteur d'hydroformylation.

18. Procédé selon la revendication 17, **caractérisé en ce que** du tris(2,4-ditert.-butylphényl)phosphite est utilisé en tant que composé d'organophosphore.
